(19) Europäisches Patentamt
European Patent Office
Office européen des brevets

(11) **EP 4 321 152 A1**

(12) **EUROPEAN PATENT APPLICATION**
published in accordance with Art. 153(4) EPC

(43) Date of publication:
**14.02.2024 Bulletin 2024/07**

(21) Application number: **22784809.0**

(22) Date of filing: **21.03.2022**

(51) International Patent Classification (IPC):
**A61K 9/08** (2006.01)    **A61K 9/06** (2006.01)
**A61K 9/00** (2006.01)    **A61K 47/12** (2006.01)
**A61K 47/28** (2006.01)    **A61K 47/26** (2006.01)
**A61K 47/44** (2017.01)    **A61K 31/24** (2006.01)
**A61K 38/26** (2006.01)

(52) Cooperative Patent Classification (CPC):
**A61K 9/00; A61K 9/06; A61K 9/08; A61K 31/24;
A61K 38/26; A61K 47/12; A61K 47/26;
A61K 47/28; A61K 47/44**

(86) International application number:
**PCT/KR2022/003912**

(87) International publication number:
**WO 2022/215899 (13.10.2022 Gazette 2022/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **08.04.2021   KR 20210045906**

(71) Applicant: **Tionlab Therapeutics
Gyeonggi-do 17028 (KR)**

(72) Inventors:
• **LIM, Duck Soo**
  **Suwon-si Gyeonggi-do 16628 (KR)**
• **LEE, Eun Joo**
  **Hwaseong-si Gyeonggi-do 18452 (KR)**

(74) Representative: **Schiweck Weinzierl Koch
Patentanwälte Partnerschaft mbB
Ganghoferstraße 68 B
80339 München (DE)**

(54) **SUSTAINED-RELEASE LIPID PRE-CONCENTRATE**

(57)    The present invention relates to a sustained-release lipid pre-concentrate. The sustained-release lipid pre-concentrate according to the present invention forms liquid crystals when exposed to an aqueous medium, thus enabling the sustained-release of a drug, and can sustain drug release for two weeks to one month. In addition, the formed liquid crystals are sustainably released even at low viscosity, and have excellent ease of use due to low extrusion force when injected.

[FIG. 1]

Preparation Example 1-1

Preparation Example 1-2

Preparation Example 1-3

Preparation Example 1-4

**Description**

[Technical Field]

The present invention relates to a sustained-release lipid pre-concentrate.

[Background Art]

A sustained-release concentrate is a formulation that can provide a sustained release of a pharmacologically active material with a single dose to prevent side effects that may occur upon repeated doses and maintain the effective concentration range of the pharmacologically active material for a certain time or a certain period of time.

A representative sustained-release material that is biodegradable and currently in use is poly(lactic-co-glycolic acid) (PLGA), which has been approved by the Food and Drug Administration (FDA). U.S. Patent No. 5,480,656 discloses that a PLGA biodegradable polymer can be decomposed into lactic acid and glycolic acid after a certain period of time *in vivo,* allowing the sustained release of a pharmacologically active material. However, acidic materials that are decomposition products of PLGA can trigger an inflammatory response and reduce cell proliferation rate (K. Athanasiou, G G Niederauer, and C. M. Agrawal, Biomaterials, 17, 93 (1996)). Further, for sustained release, a drug needs to be encapsulated in PLGA solid particles with a size of approximately 10 to 100 micrometers and then injected, but in this case, there is a problem with pain or inflammation upon injection. Therefore, there is a need for the development of a new sustained release formulation that can increase patient medication compliance while providing the effective concentration of a pharmacologically active material for a certain period of time or more.

As a formulation that can avoid the disadvantages of biodegradable polymer-based formulations, WO 2005/117830 discloses a liquid depot formulation including: at least one neutral diacylipid (for example, a diacyl glycerol such as glyceryl diolate) and/or at least one tocopherol; at least one phospholipid; and at least one low-viscosity organic solvent containing biocompatible oxygen. However, since formulations containing a neutral diacyl lipid such as glyceryl diolate have a problem of low biodegradability and are not bio-derived materials, they have limited biocompatibiliy and are highly likely to induce inflammation.

In addition, Korean Patent No. 10-1494594 discloses a sustained-release lipid pre-concentrate including a liquid crystal hardener whose hydrophobic moiety has a triacyl group having 15 to 40 carbon atoms or a carbon ring structure without having a sorbitan unsaturated fatty acid ester; a phospholipid such as phosphatidyl choline; and an ionized group of a carboxylic group or an amine group. However, since sorbitan monooleate has a high viscosity (about 2000 mPa.s at 25°C), formulations obtained using sorbitan monooleate also have a high viscosity, and thus have a problem of low injection force (injectability).

Therefore, there is a need to develop a sustained-release pharmaceutical composition for injection in the art, which can prevent the initial drug release phenomenon and has excellent biodegradability, biocompatibiliy, and injection power as a long-lasting drug injection preparation for two weeks or more.

[Related Art Document]

[Patent Document]

1. International Publication No. WO 2005/117830

2. Korean Patent No. 10-1494594

[Disclosure]

[Technical Problem]

The present invention relates to a sustained-release lipid pre-concentrate in the form of a lipid solution, and an object thereof is to provide a sustained-release lipid pre-concentrate that spontaneously undergoes a phase transition and forms liquid crystals when exposed to an aqueous medium.

Furthermore, an object of the present invention is to provide a sustained-release lipid pre-concentrate that is sustainably released even at low viscosity, and has excellent viscoelasticity, excellent ease of use due to low extrusion force when injected, and a sustainability of 2 weeks to 1 month.

[Technical Solution]

The present invention provides a sustained-release lipid pre-concentrate, which forms liquid crystals in an aqueous medium, including: a) a vegetable oil including an unsaturated fatty acid having 14 to 20 carbon atoms; and b) one or more compounds selected from the group consisting of cholesterol and a sugar alcohol.

[Advantageous Effects]

The sustained-release lipid pre-concentrate according to the present invention forms liquid crystals when exposed to an aqueous medium, thus enabling the sustained-release of a drug, and can sustain drug release for two weeks to one month. Furthermore, liquid crystals formed by the sustained-release lipid pre-concentrate are sustainably released even at low viscosity, and have excellent viscoelasticity, and excellent ease of use due to low extrusion force when injected.

[Description of Drawings]

FIG 1 shows a set of photographs of sustained-release lipid pre-concentrates prepared in Preparation Example 1 being injected into PBS at pH 7.4.
FIG 2A and 2B shows a set of photographs of sustained-release lipid pre-concentrates prepared in Preparation Example 2 being injected into PBS at pH 7.4 and then shaken for 3 to 5 seconds.
FIG 3A and 3B shows a set of photographs of sustained-release lipid pre-concentrates prepared in Preparation Example 3 being injected into PBS at pH 7.4.
FIG 4 shows a photograph of sustained-release lipid pre-concentrates prepared in Preparation Example 4 being injected into PBS at pH 7.4.
FIG 5 shows the results of evaluating the peroxide value of the liquid crystal former according to the type of stabilizer.
FIG 6 shows the results of evaluating the injection force of sustained-release lipid pre-concentrates prepared in Preparation Example 5.
FIG 7 shows the results of evaluating the elution of sustained-release lipid pre-concentrates prepared in Preparation Example 5.
FIG 8A and 8B shows a photograph of sustained-release lipid pre-concentrates prepared in Preparation Example 6 and a photograph of the sustained-release lipid pre-concentrates when injected into PBS at pH 7.4.
FIG 9A and 9B shows a set of photographs of sustained-release lipid pre-concentrates prepared in Preparation Example 7 being injected into PBS at pH 7.4.
FIG 10 shows the results of evaluating the elution of sustained-release lipid pre-concentrates prepared in Preparation Example 8.
FIG 11 shows changes in concentration of semaglutide over time.

[Modes of the Invention]

Hereinafter, the present invention will be described in detail.
However, the present invention may be modified in various forms and may have various forms, so that specific examples and descriptions set forth below are included merely for aiding the understanding of the present invention, and are not intended to limit the present invention to a specific disclosure form. It should be understood that the scope of the present invention includes all the modifications, equivalents, and replacements falling within the spirit and technical scope of the present invention.
The present invention relates to a sustained-release lipid pre-concentrate, which forms liquid crystals in an aqueous medium, including: a) a vegetable oil including an unsaturated fatty acid having 14 to 20 carbon atoms; and b) one or more compounds selected from the group consisting of cholesterol and a sugar alcohol.
In the present invention, the pre-concentrate corresponds to a formulation that can release a drug for a long period of time, more specifically, it may be a sustained-release injection formulation or oral formulation. The sustained release formulation needs to gradually release the drug inside to an intended level over a period of time. In the present invention, the pre-concentrate is in the form of a pre-formulated lipid solution, and forms a non-lamellar phase, specifically non-lamellar liquid crystals (hereinafter referred to as liquid crystals) when exposed to an aqueous medium such as water or a biological fluid.
In a specific embodiment, the sustained-release lipid pre-concentrate may have a viscosity of 30 to 650 cp. When the pre-concentrate has the above viscosity, it can be injected, may form a gel that is sustainable in the body, and may also be expressed as the formation of strong liquid crystals.

In the present invention, the sustained-release lipid pre-concentrate includes a) a vegetable oil including an unsaturated fatty acid having 14 to 20 carbon atoms.

Component a) may act as a liquid crystal former. That is, the vegetable oil may form liquid crystals as a liquid crystal former in an aqueous medium.

In a specific embodiment, the unsaturated fatty acid having 14 to 20 carbon atoms is an unsaturated fatty acid having one or two double bonds, and may have excellent biocompatibiliy and biodegradability. Such unsaturated fatty acids may provide excellent injection force (injectability) by rapidly forming a low-viscosity lipid solution. That is, the pre-concentrate of the present invention may form strong liquid crystals when brought into contact with an aqueous medium. Furthermore, since the pre-concentrate of the present invention has a low viscosity at room temperature (about 25°C), it can be easily introduced into a living body through a syringe of 18 gauge or higher.

In a specific embodiment, the type of component a) is not particularly limited, and may be one or more selected from the group consisting of, for example, castor oil, safflower oil, cottonseed oil, almond oil, avocado oil, canola oil, coconut oil, corn oil, flaxseed oil, linseed oil, macadamia oil, mustard oil, olive oil, palm oil, peanut oil, pumpkin seed oil, rice bran oil, sesame oil, soybean oil, sunflower oil, tea seed oil and walnut oil. Specifically, in the present invention, a mixture of castor oil and safflower oil or a mixture of castor oil and cottonseed oil may be used as component a).

In a specific embodiment, the content of component a) is not particularly limited, and component a) may be included in an amount of 50 to 98 parts by weight based on the total weight (100 parts by weight) of the pre-concentrate. When the content of component a) is less than 50 parts by weight or more than 98 parts by weight, the ability to form liquid crystals remarkably deteriorates and the ability to sustainably release an active ingredient may deteriorate when the sustained-release lipid pre-concentrate according to the present invention is injected *in vivo.* As a result, there is concern that the ability to control release for two weeks or more, which is the object of the present invention, may not be exhibited.

In the present invention, the sustained-release lipid pre-concentrate includes b) one or more compounds selected from the group consisting of cholesterol and a sugar alcohol.

Component b) may act as a liquid crystal hardener. That is, component b) may aid in stiffening the internal structure of the liquid crystal and may serve to delay the release rate of an active ingredient.

In a specific embodiment, the sugar alcohol may include one or more selected from the group consisting of mannitol, sorbitol, xylitol, erythritol, maltitol and lactitol.

In a specific embodiment, the content of component b) is not particularly limited, and component b) may be included in an amount of 2 to 50 parts by weight based on the total weight (100 parts by weight) of the pre-concentrate.

In a specific embodiment, the content of component b) may be 1 to 10 parts by weight or 2 to 5 parts by weight based on 100 parts by weight of component a). In the above content range, the internal structure of the liquid crystal may be strongly maintained.

The sustained-release lipid pre-concentrate of the present invention may further include an active ingredient. The active ingredient is used for release purposes and may preferably be a drug to treat a particular disorder, symptom or disease.

In a specific embodiment, the drug may include an antibacterial agent such as a β-lactam or macrocyclic peptide antibiotic, an antifungal agent such as a polyene macrolide (for example, amphotericin B) or an azole antibacterial agent, a nucleoside analogue, an anti-cancer agent such as paclitaxel and derivatives thereof and/or an antiviral agent, an anti-inflammatory agent such as a non-steroidal anti-inflammatory agent, a therapeutic agent for pancreatitis such as nafamostat, a cardiovascular agent including a cholesterol-lowering agent and an antihypertensive, an analgesic, an anesthetic agent, an antidepressant including a serotonin absorption inhibitor, a vaccine, and a bone regulator. Alternatively, the drug may include insulin and insulin analogues, growth hormones such as human growth hormone (hgh) immunosuppressants such as tacrolimus and cyclosporin A, peptide drugs such as octreotide, salmon calcitonin, desmopressin, somatostatin, antibodies and antibody fragments, nucleic acids including antisense and interfering nucleic acids (for example, siRNAs) and vaccines.

Specifically, the drug may be one or more selected from the group consisting of nafamostat, doxycycline, liraglutide, semaglutide, lanreotide and goserelin.

In a specific embodiment, the content of the drug is not particularly limited, and may be 1 to 80 parts by weight or 10 to 30 parts by weight based on 100 parts by weight of component a). In the above content range, the structure of the liquid crystal is not affected, and the drug can be sustainably released for 2 weeks to 1 month.

The sustained-release lipid pre-concentrate may further include a stabilizer. The stabilizer aids in maintaining the internal structure of the liquid crystals, and can produce strong liquid crystals. Further, the stabilizer can serve to reduce the viscosity of the sustained-release pre-concentrate.

In a specific embodiment, the type of stabilizer is not particularly limited, and may be one or more selected from the group consisting of, for example, sodium ascorbate (ViC_Na), ascorbic palmitate (ViC_palmitate), butylated hydroxyanisole (BHA), butylated hydroxytoluene (BHT), methionine, monothioglycerol, sodium thiosulfate and sodium metabisulfite.

In a specific embodiment, the content of the stabilizer is not particularly limited, and may be 0.1 to 10 parts by weight based on 100 parts by weight of component a).

The sustained-release lipid pre-concentrate may further include a solvent. The solvent may serve to improve the ability to dissolve or inject the main component. When a sustained-release lipid pre-concentrate including a solvent is injected *in vivo,* the solvent may be diluted by a biofluid during the production of liquid crystals and removed.

In a specific embodiment, the type of solvent is not particularly limited, and any solvent capable of being introduced into the human body in the form of an injection may be used. For example, an organic solvent selected from the group consisting of ethanol (EtOH), propylene glycol, N-methylpyrrolidone (NMP), and benzyl alcohol or an aqueous solution of the organic solvent may be used.

In a specific embodiment, the solvent may be included in an amount of 1 to 30 parts by weight based on the total weight (100 parts by weight) of the sustained release pre-concentrate.

The sustained-release lipid pre-concentrate may further include a surfactant. The surfactant may serve to improve the dissolution of the main component.

In a specific embodiment, the type of surfactant is not particularly limited, and it is possible to use one or more selected from the group consisting of, for example, Tween, cremophor, solutol, Brij, Triton, a sugar fatty acid ester (for example, sucrose, oleate, sucrose palmitate and/or sucrose laurate), and a surfactant having a polyoxyethylene head group.

In a specific embodiment, the surfactant may be included in an amount of 0.1 to 10 parts by weight based on the total weight (100 parts by weight) of the sustained release pre-concentrate.

The sustained-release lipid pre-concentrate may not include a diacyl glycerol and a sorbitan unsaturated fatty acid ester. The acyl glycerol may be glyceryl dipalmitate, glyceryl phytanoate, glyceryl palmitoleate, glyceryl distearate, glyceryl dioleate, glyceryl dielaidiate, glyceryl dilinoleate, and the like, and the sorbitan unsaturated fatty acid ester may be sorbitan monooleate, sorbitan monolinoleate, sorbitan monopalmitoleate, sorbitan monomyristoleate, sorbitan sesquioleate, sorbitan sesquilinoleate, sorbitan sesquipalmitoleate, sorbitan sesquimyristoleate, sorbitan dioleate, sorbitan dilinoleate, sorbitan dipalmitoleate, sorbitan dimyristoleate, and the like.

In addition, the sustained-release lipid pre-concentrate of the present invention may not include tocopherol.

The sustained-release lipid pre-concentrate according to the present invention may have the following physicochemical characteristics.

(i) Viscosity is 30 to 650 cp, and
(ii) injection force is 1 to 50 N.

Viscosity is a quantity that represents the magnitude of the viscous property meaning the resistance to fluid flow. The higher the viscosity, the greater the injection force, so it is better to have an appropriate viscosity. In the present invention, viscosity may be measured under the conditions of the experimental examples to be described below using DV-II+Pro manufactured by Brookfield.

In the present invention, the sustained-release lipid pre-concentrate may have a viscosity of 30 to 650 cP. The sustained-release lipid pre-concentrate can be used as an injection in the above viscosity range, and may form strong liquid crystals in an aqueous medium.

Injection force (injectability) is the extrusion force at an injection rate that is comfortable for a patient. "Comfortable for a patient" is used to define an injection rate that does not cause injury or undue pain to the patient during injection. "Comfort" as used in the present invention includes not only the comfort of a patient, but also the comfort or ability of a doctor or medical professional to inject a composition. In the present invention, injection force may be measured under the conditions of the experimental examples to be described below using 5569 equipment manufactured by INSTRONS. In general, a composition with low injection force causes no tenderness during injection and is easy to control.

In the present invention, the sustained-release lipid pre-concentrate may have an injection force of 1 to 50 N or 1 to 12.5 N.

Furthermore, the present invention relates to a method for preparing the above-described sustained-release lipid pre-concentrate.

In the present invention, a sustained-release lipid pre-concentrate may be prepared through heating a mixture of component a) and component b) (hereinafter referred to as a heating step); and cooling the mixture (hereinafter referred to as a cooling step).

In a specific embodiment, the heating temperature in the heating step is not particularly limited as long as component a) and component b) can be completely dissolved or homogenized, and may be, for example, 40 to 70°C or 45 to 60°C.

In a specific embodiment, one or more components selected from the group consisting of a solvent, a stabilizer and a surfactant may be mixed together at the time of mixing of component a) and component b).

In a specific embodiment, the drug may be mixed together at the time of mixing component a) and component b), or the drug may be mixed after the cooling step is performed.

In a specific embodiment, the cooling step may be performed at 20 to 30°C or room temperature.

Further, the present invention relates to a sustained-release pharmaceutical composition for injection in the form of a lipid solution including the above-described lipid pre-concentrate.

In a specific embodiment, the sustained-release pharmaceutical composition may be used for subcutaneous or intramuscular injection.

In addition, the present invention relates to an oral pharmaceutical composition including the above-described sustained-release lipid pre-concentrate.

[Mode for Invention]

Hereinafter, the present invention will be described in detail through the Examples. However, the following examples are only for exemplifying the present invention, and the scope of the present invention is not limited to the following Examples. The present examples are provided to make the disclosure of the present invention complete and to allow a person skilled in the art to which the present invention belongs to completely comprehend the scope of the present invention, and the present invention is defined only by the scope of the claims.

Examples

**[Reference] Materials**

As vegetable oils, Super Refined Castor oil-LQ-(MH), Super Refined Safflower USP-LQ-(MH), Super Refined Cottonseed NF, Super Refined Sesame NF-LQ-(MH), and the like manufactured by Croda were used.

Furthermore, Cholesterol and plant-derived (USP) manufactured by SAFC, D-mannitol (USP) and isomeric-GDO (1,3-GDO) manufactured by Merck, and ascorbic acid-6-palmitate, sodium ascorbate, soy-PC, DL-α--tocopherol acetate, Span80, and oleic acid manufactured by Sigma were used.

**Preparation Example 1. Preparation of sustained-release lipid pre-concentrate including nafamostat as drug**

Sustained-release lipid pre-concentrates including nafamostat as a drug were prepared according to the components and contents in the following Table 1.

Specifically, the components shown in the following Table 1 were added to a glass vial, and then mixed while stirring with a magnetic stirrer at 45 to 60°C. After the drug was added to the obtained mixture at room temperature, the resulting mixture was homogenized under the condition of about 10,000 rpm for about 5 minutes using a homogenizer (POLYTRON PT1200E, KINEMATICA), and then left to stand at room temperature for about 3 hours to prepare a sustained-release lipid pre-concentrate.

[Table 1]

|  | Preparation Example 1-1 | Preparation Example 1-2 | Preparation Example 1-3 | Preparation Example 1-4 |
|---|---|---|---|---|
| Nafamostat (g) | 2.25 | 2.25 | 2.25 | 2.25 |
| Cholesterol (mg) | 300 | 0 | 0 | 0 |
| D-mannitol (mg) | 0 | 300 | 0 | 0 |
| Phosphatidylcholine (PC) (mg) | 0 | 0 | 300 | 300 |
| Castor oil (g) | 3 | 3 | 0 | 0 |
| Safflower oil (g) | 1.5 | 1.5 | 0 | 0 |
| Span80 (g) | 0 | 0 | 4.5 | 0 |
| Oleic acid (g) | 0 | 0 | 0 | 4.5 g |

**Test Example 1. Evaluation of ability of sustained-release lipid pre-concentrate including nafamostat as drug to form liquid crystals in aqueous phase**

The sustained-release lipid pre-concentrate prepared in Preparation Example 1 was filled into a 1-ml syringe and injected into 5 ml of a phosphate buffer (PBS) with pH 7.4 through a 23 gauge injection needle.

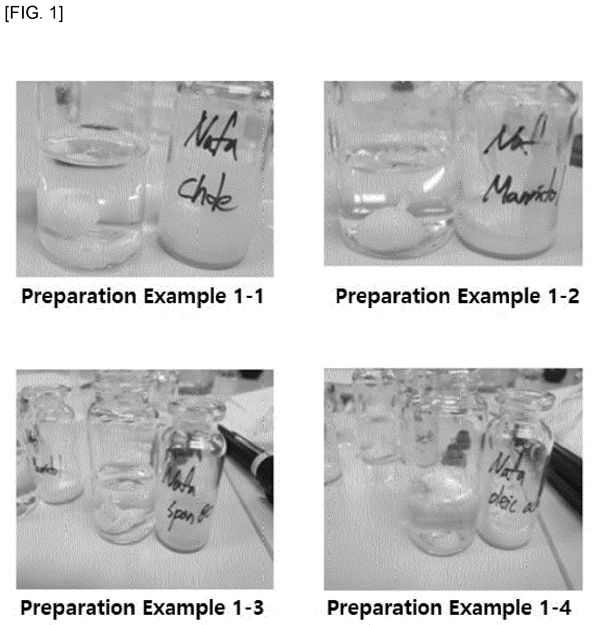

FIG 1 shows a set of photographs of sustained-release lipid pre-concentrates prepared in Preparation Example 1 being injected into PBS at pH 7.4.

As shown in FIG 1, it can be confirmed that when cholesterol, castor oil and safflower oil were used together (Preparation Example 1-1), or when mannitol, castor oil and safflower oil were used together (Preparation Example 1-2), liquid crystals were formed in the aqueous phase. The liquid crystals formed in the above preparation examples may have sustainability from 2 weeks to 1 month or more.

In contrast, it can be confirmed that no liquid crystals were formed in Preparation Examples 1-3 and 1-4 in which phosphatidylcholine and span80 or phosphatidylcholine and oleic acid were used.

**Preparation Example 2. Preparation of sustained-release lipid pre-concentrate according to cholesterol content**

Sustained-release lipid pre-concentrates were prepared according to the components and contents of the following Table 2.

The sustained-release lipid pre-concentrate was prepared as in the preparation method of Preparation Example 1.

[Table 2]

|  | Preparation Example 2-1 | Preparation Example 2-2 | Preparation Example 2-3 | Preparation Example 2-4 | Preparation Example 2-5 |
|---|---|---|---|---|---|
| Nafamostat (g) | 2.25 | 2.25 | 2.25 | 2.25 | 2.25 |
| Cholesterol (mg) | 0 | 50 | 150 | 200 | 250 |
| Castor oil (g) | 3 | 3 | 3 | 3 | 3 |
| Safflower oil (g) | 1.5 | 1.5 | 1.5 | 1.5 | 1.5 |

**Test Example 2. Evaluation of ability of sustained-release lipid pre-concentrate to form liquid crystals in aqueous phase according to cholesterol content**

The sustained-release lipid pre-concentrate prepared in Preparation Example 2 was filled into a 1-ml syringe and injected into 5 ml of a phosphate buffer (PBS) with pH 7.4 through a 23 gauge injection needle.

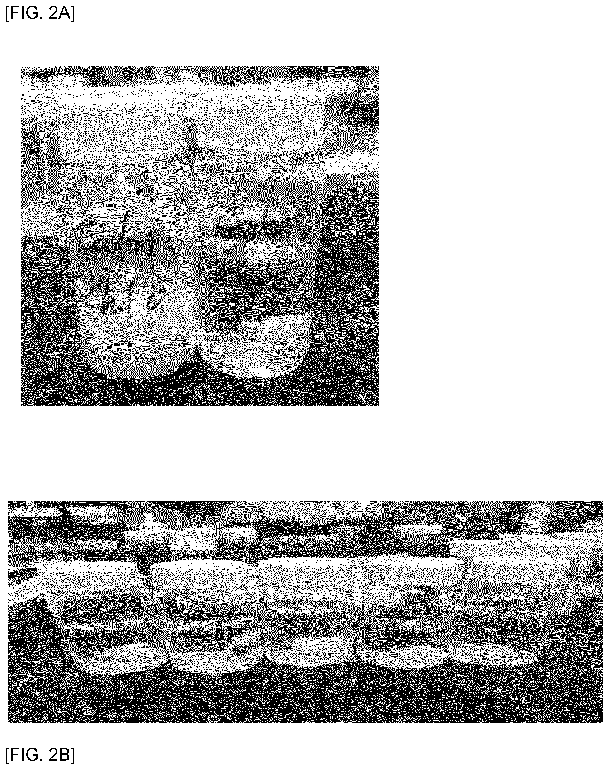

FIG 2A is a photograph of the sustained-release lipid pre-concentrate of Preparation Example 2-1 after injection into PBS with pH 7.4, and it can be confirmed that liquid crystals are formed in the aqueous phase even when cholesterol is not contained, but the strength of the liquid crystals is very weak.

Further, FIG 2B shows a photograph of the sustained-release lipid pre-concentrate prepared in Preparation Example 2 injected into PBS at pH 7.4 and then shaken for 3 to 5 seconds. Preparation Examples 2-1, 2-2, 2-3, 2-4 and 2-5 are shown from the left container in FIG 2B.

As shown in FIG 2B, it could be confirmed that the sustained-release lipid pre-concentrate of Preparation Example 2-1 could not maintain the structure of liquid crystals and was decomposed when injected into PBS, and then shaken.

In this regard, it can be confirmed that when cholesterol is included, the structure of liquid crystals is maintained even when the sustained-release lipid pre-concentrate is injected into PBS, and then shaken, and in particular, when the content of cholesterol is 150 mg or more, formulation stability was excellent.

**Preparation Example 3. Preparation of sustained-release lipid pre-concentrate further including stabilizer**

Sustained-release lipid pre-concentrates were prepared according to the components and contents of the following Table 3.

The sustained-release lipid pre-concentrate was prepared as in the preparation method of Preparation Example

1.

[Table 3]

|  | Preparation Example 3-1 | Preparation Example 3-2 | Preparation Example 3-3 | Preparation Example 3-4 | Preparation Example 3-5 | Preparation Example 3-6 |
|---|---|---|---|---|---|---|
| Nafamostat (g) | 2.25 | 2.25 | 2.25 | 2.25 | 2.25 | 2.25 |
| Cholesterol (mg) | 200 | 100 | 100 | 0 | 0 | 200 |
| Tocopherol (mg) | 0 | 0 | 0 | 200 | 0 | 0 |
| Castor oil (g) | 3 | 3 | 3 | 3 | 3 | 1.5 |
| Safflower oil (g) | 1.5 | 1.5 | 1.5 | 1.5 | 1.5 | 3 |
| Ascorbic palmitate (mg) | 0 | 0 | 50 | 0 | 200 | 0 |

**Test Example 3. Evaluation of ability of sustained-release lipid pre-concentrate to form liquid crystals in aqueous phase according to use of stabilizer**

The sustained-release lipid pre-concentrate prepared in Preparation Example 3 was filled into a 1-ml syringe and injected into 5 ml of a phosphate buffer (PBS) with pH 7.4 through a 23 gauge injection needle.

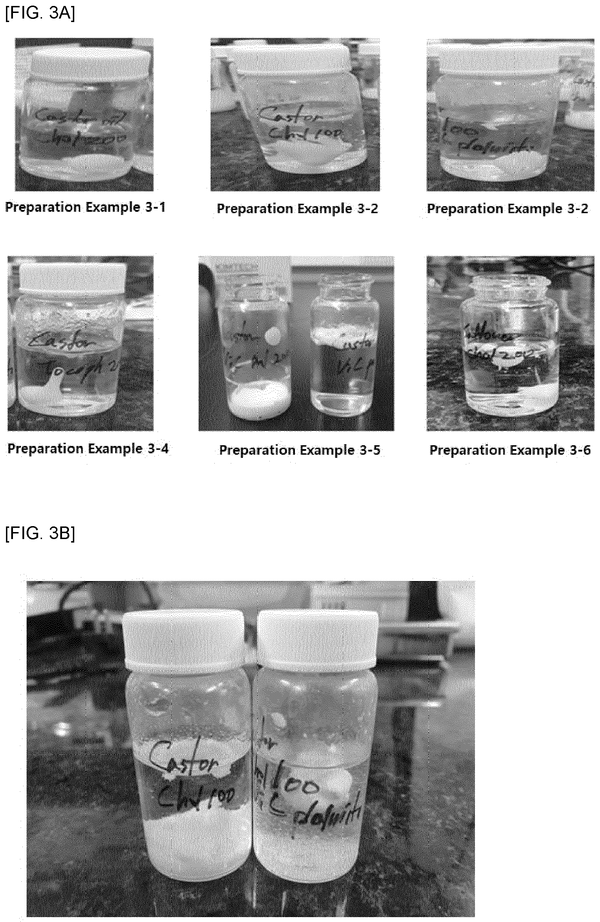

FIG 3A shows a set of photographs of sustained-release lipid pre-concentrates prepared in Preparation Example 3 being injected into PBS at pH 7.4.

As shown in FIG 3A, it can be confirmed that liquid crystals were formed when cholesterol and a vegetable oil were included together (Preparation Examples 3-1 to 3-3 and 3-6). However, it can be confirmed that when the content of cholesterol was 100 mg, the formed liquid crystals had a weak strength, but when ascorbic palmitate was additionally included as a stabilizer, it was possible to produce liquid crystals that were strong enough to include 200 mg of cholesterol and had excellent formulation stability even though the content of cholesterol was 100 mg.

Meanwhile, it can be confirmed that when tocopherol was used instead of using cholesterol (Preparation Example 3-4), liquid crystals were formed, but liquid crystals having weak strength were formed, and that when ascorbic palmitate was used (Preparation Example 3-5), liquid crystals were not formed.

In addition, FIG 3B shows a photograph of liquid crystal morphologies observed 45 days after injecting sustained-release lipid pre-concentrates of Preparation Examples 3-2 (left container) and 3-3 (right container) into PBS with pH 7.4.

As shown in FIG 3B, it can be confirmed that Preparation Example 3-3 in which ascorbic palmitate was added as a stabilizer had excellent formulation stability for a long period of time of one month or more without affecting viscosity.

**Preparation Example 4. Preparation of sustained-release lipid pre-concentrate according to type of liquid crystal former**

Sustained-release lipid pre-concentrates were prepared according to the components and contents of the following Table 4.

The sustained-release lipid pre-concentrate was prepared as in the preparation method of Preparation Example 1.

[Table 4]

|  | Preparation Example 4-1 | Preparation Example 4-2 | Preparation Example 4-3 | Preparation Example 4-4 |
|---|---|---|---|---|
| Nafamostat (g) | 2.25 | 1.5 | 1.5 | 1.5 |
| Cholesterol (mg) | 200 | 200 | 200 | 200 |

(continued)

|  | Preparation Example 4-1 | Preparation Example 4-2 | Preparation Example 4-3 | Preparation Example 4-4 |
|---|---|---|---|---|
| Castor oil (g) | 3 | 0 | 0 | 0 |
| Safflower oil (g) | 1.5 | 0 | 0 | 0 |
| Span80 (g) | 0 | 3 | 0 | 0 |
| Oleic acid (g) | 0 | 0 | 3 | 0 |
| Isomeric GDO (g) | 0 | 0 | 0 | 3 |

**Test Example 4. Evaluation of ability of sustained-release lipid pre-concentrate to form liquid crystals in aqueous phase according to type of liquid crystal former**

The sustained-release lipid pre-concentrate prepared in Preparation Example 4 was filled into a 1-ml syringe and injected into 5 ml of a phosphate buffer (PBS) with pH 7.4 through a 23 gauge injection needle.

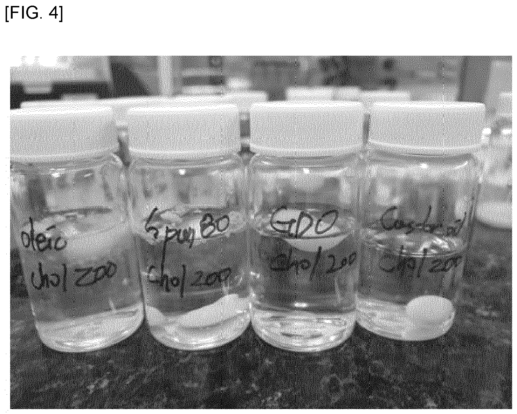

FIG 4 shows a photograph of sustained-release lipid pre-concentrates prepared in Preparation Example 4 being injected into PBS at pH 7.4.

As shown in FIG 4, it can be confirmed that strong liquid crystals were formed in Preparation Example 4-1 (rightmost container) using the mixture of castor oil and safflower oil according to the present invention as a liquid crystal former.

However, it can be confirmed that Preparation Example 4-2 (second container from the left) using Span 80 is not mixed with the drug and cholesterol, Preparation Example 4-3 (leftmost container) using oleic acid does not form liquid crystals, and Preparation Example 4-4 (second container from the right) using Isomeric GOD becomes hard over time.

**Test Example 5. Evaluation of peroxide value of liquid crystal former according to type of stabilizer**

0.3 g of each of tocopherol, ViC_palmitate, mannitol, and ViC_Na was added as stabilizers to 3 g of each of safflower oil, castor oil, cottonseed oil, and oleic acid. After the resulting mixture was heated at 45 to 60°C after DI treatment, it was cooled to room temperature and left to stand for 3 hours.

The degree of rancidity was evaluated by allowing the mixture to stand under harsh conditions of 60°C for two weeks to measure the peroxide value of the liquid crystal former.

In order to measure the peroxide value, 0.3 g of the sample was weighed into a 300-ml Erlenmeyer flask, and then 30 ml of a solution of chloroform: glacial acetic acid (2:3) was added thereto. After a temporary dark place was created with silver foil paper, 1 ml of a KI saturated solution was added, and the resulting mixture was shaken vigorously, and then left to stand in the dark place for 5 minutes. Thereafter, 30 ml of distilled water was again added thereto, 1 ml of a 1% starch solution was added thereto, and then the resulting mixture was vigorously mixed. Thereafter, the mixture was titrated with a 0.01 N-$Na_2S_2O_3$ standard solution while stirring with a magnetic stirrer, and the end point was set to the time when the dark blue color of the starch disappeared. Thereafter, the peroxide value of the sample was determined using the following equation.

$$\text{Peroxide value (meq/kg)} = \frac{(A-B) \times Fr}{Sr} \times 10$$

A= Consumed amount (ml) of 0.01N-$Na_2S_2O_3$ during the titration in this test
B = Consumed amount (ml) of 0.01N- $Na_2S_2O_3$ during the titration in a blank test
Sr = Weight (g) of the sample
Fr = 0.01N- $Na_2S_2O_3$ titer

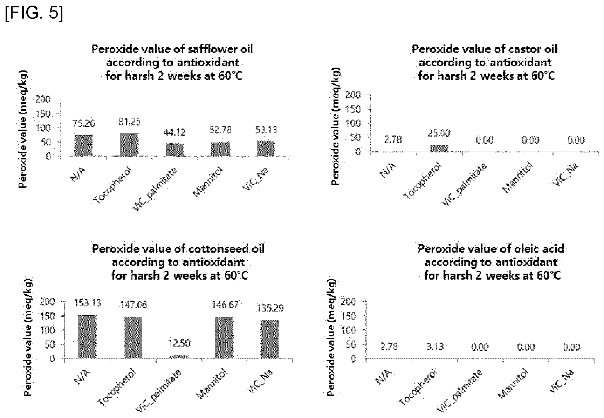

FIG 5 shows the results of evaluating the peroxide values of liquid crystal formers depending on the type of stabilizer.

As shown in FIG 5, it can be confirmed that when ViC_palmitate was used, the peroxide values of all the liquid crystal formers used in this test example were low. This suggests that when ViC_palmitate is used as a stabilizer, liquid crystals having excellent formulation stability can be produced.

Meanwhile, it can be confirmed that when tocopherol is used as a stabilizer, the peroxide value is higher than when no stabilizer is used. Therefore, it is better not to use tocopherol as a stabilizer.

**Preparation Example 5. Preparation of sustained-release lipid pre-concentrate including nafamostat as a drug 2**

Sustained-release lipid pre-concentrates were prepared according to the components and contents of the following Table 5.

The sustained-release lipid pre-concentrate was prepared as in the preparation method of Preparation Example 1.

[Table 5]

|  | Preparation Example 5-1 | Preparation Example 5-2 | Preparation Example 5-3 | Preparation Example 5-4 |
|---|---|---|---|---|
| Nafamostat (g) | 1.5 | 1.5 | 2.25 | 2.25 |
| Cholesterol (mg) | 200 | 200 | 200 | 200 |
| Oleic acid | 3 | 0 | 0 | 0 |
| Span80 | 0 | 3 | 0 | 0 |
| Castor oil | 0 | 0 | 1.5 | 3 |
| Safflower oil | 0 | 0 | 3 | 1.5 |

**Test Example 6. Evaluation of injection force (injectability)**

An 18-gauge syringe including the contents was attached to 5569 equipment manufactured by INTRONS, and the injection force was evaluated as a load (N) measured when pressurized at a speed of 100 mm per minute.

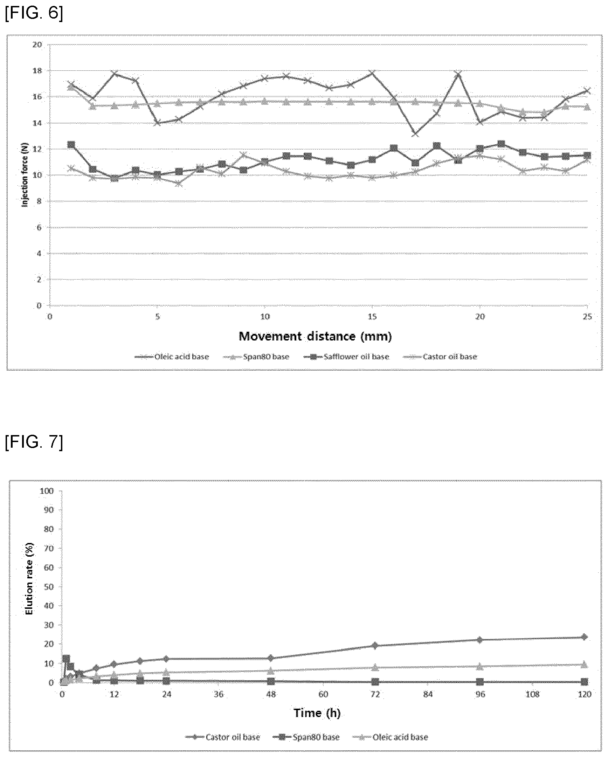

FIG 6 shows the results of evaluating the injection force of sustained-release lipid pre-concentrates prepared in Preparation Example 5.

As shown in FIG 6, it can be confirmed that the injection force is about 13 N or more when oleic acid or Span80 is used, but the sample has a low injection force of about 12.5 N or less when a vegetable oil such as castor oil or safflower oil is used as in the present invention.

**Test Example 7. Evaluation of elution**

900 ml of a phosphate buffer with pH 7.4 was put into an elutor, and elution was performed while rotating the elutor at 50 rpm for 120 hours using the USP I basket method.

FIG 7 shows the results of evaluating the elution of sustained-release lipid pre-concentrates prepared in Preparation Example 5.

As shown in FIG 7, it can be confirmed that continuous elution is shown when castor oil is used.

When Span80 was used, a rapid elution rate was shown at the initial stage, and the sample settled into the vessel of the elutor and became hard while forming a gel (liquid crystals). Therefore, the elution rate is not measured. In addition, since the sustained-release lipid pre-concentrate floats on top of the effluent in the vessel of the elutor and sticks to the vessel wall without forming a gel when oleic acid is used, the elution rate is not measured.

**Test Example 8. Evaluation of viscosity according to composition of liquid crystal former**

Formulations with various compositions were prepared using castor oil, safflower oil and cottonseed oil as liquid crystal formers. In this case, a solvent (NMP) was additionally used for some formulations.

The viscosity of the formulation prepared above was measured.

The viscosity was measured while rotating a conical plate at 50 rpm using DV-II+ Pro manufactured by Brookfield.

Table 6 shows the viscosity measurement results.

[Table 6]

| Oil type | cP | |
|---|---|---|
| | 25°C | 34.5°C |
| Castor | 699.80 | 348.00 |
| Cottonseed | 50.46 | 35.16 |
| Safflower oil | 56.40 | 38.88 |

| Castor/Safflower oil | cP | | Castor/Cotton Oil | cP | |
|---|---|---|---|---|---|
| | 25°C | 34.5°C | | 25°C | 34.5°C |
| 1 :4 | 74.25 | 49.26 | 1 : 4 | 83.10 | 53.40 |
| 2 : 3 | 120.2 | 76.35 | 2 : 3 | 133.50 | 82.20 |
| 1 : 1 | 152.7 | 95.1 | 1 : 1 | 170.70 | 105.90 |
| 3:2 | 203.7 | 117.3 | 3 : 2 | 225.00 | 126.60 |
| 4: 1 | 364.2 | 201 | 4: 1 | 374.40 | 215.40 |

| Formulation | NMP 2 % | | NMP 3 % | | NMP 5 % | |
|---|---|---|---|---|---|---|
| Castor/Safflower Oil/NMP | 25°C | 34.5°C | 25°C | 34.5°C | 25°C | 34.5°C |
| Castor/Safflower oil (1:4) | 54.60 | 38.40 | 58.35 | 37.50 | 56.10 | 36.90 |
| Castor/Safflower oil (2:3) | 80.70 | 55.05 | 75.75 | 54.75 | 71.95 | 54.35 |
| Castor/Safflower oil (1:1) | 96.45 | 64.05 | 102.90 | 66.90 | 98.40 | 69.30 |
| Castor/Safflower oil (3:2) | 124.80 | 77.85 | 121.50 | 84.90 | 120.90 | 88.50 |
| Castor/Safflower oil (4:1) | 140.00 | 89.85 | 203.40 | 116.40 | 193.20 | 114.90 |

As shown in the table above, it can be confirmed that when one or more selected from the group consisting of castor oil, safflower oil, and cottonseed oil are used as a liquid crystal former, the sustained-release lipid pre-concentrate has a viscosity of 650 cP or less at 34.5°C.

Furthermore, it can be confirmed that even though a solvent is added, the sustained-release lipid pre-concentrate can be used as a formulation for injection.

**Preparation Example 6. Preparation of sustained-release lipid pre-concentrate including solvent**

Sustained-release lipid pre-concentrates were prepared according to the components and contents of the following Table 7.

The sustained-release lipid pre-concentrate was prepared as in the preparation method of Preparation Example 1.

[Table 7]

| | CCS-0% | CCS-5% | CCS-10% | CCS-15% |
|---|---|---|---|---|
| Cholesterol (mg) | 100 | 100 | 100 | 100 |
| Castor oil (g) | 2 | 2 | 2 | 2 |
| Cottonseed oil (g) | 3 | 3 | 3 | 3 |
| Ethanol (EtOH) (g) | 0 | 0.25 | 0.5 | 0.75 |

**Test Example 9. Evaluation of viscosity and ability of sustained-release lipid pre-concentrate to form liquid crystals in aqueous phase according to use of stabilizer**

The sustained-release lipid pre-concentrate prepared in Preparation Example 6 was filled into a 1-ml syringe and injected into 5 ml of a phosphate buffer (PBS) with pH 7.4 through a 23 gauge injection needle.

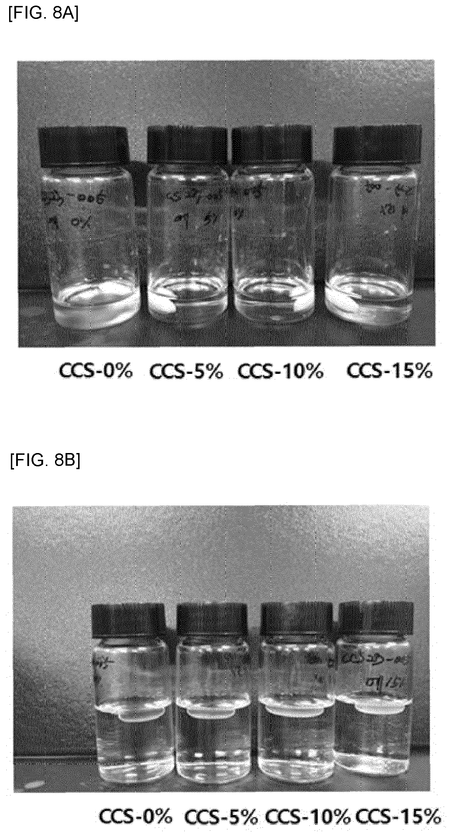

FIG 8 shows a photograph (A) of sustained-release lipid pre-concentrates prepared in Preparation Example 6 and a photograph (B) of the sustained-release lipid pre-concentrates when injected into PBS at pH 7.4.

As shown in FIG 8, it can be confirmed that liquid crystals are formed even when a solvent is used in the same manner as when no solvent is used, during the preparation of the sustained-release lipid pre-concentrate.

Meanwhile, the following FIG 8 shows the results of evaluating the viscosity of sustained-release lipid pre-concentrates prepared in Preparation Example 6.

[Table 8]

|  | CCS-0% | CCS-5% | CCS-10% | CCS-15% |
|---|---|---|---|---|
| 25°C | 137.5 | 113.3 | 66.2 | 50.2 |
| 34.5°C | 90.3 | 62 | 45.78 | 32.5 |

As shown in Table 8, it can be confirmed that the sustained-release lipid pre-concentrate prepared in Preparation Example 6 has a viscosity of 650 cP or less, and thus can be used as a formulation for injection.

**Preparation Example 7. Preparation of sustained-release lipid pre-concentrate including goserelin as drug**

Sustained-release lipid pre-concentrates including goserelin as a drug were prepared according to the components and contents in the following Table 9.

The sustained-release lipid pre-concentrate was prepared as in the preparation method of Preparation Example 1.

[Table 9]

|  | CCG-0% | CCG-5% | CCG-10% | CCG-15% |
|---|---|---|---|---|
| Goserelin (g) | 0.1 | 0.1 | 0.1 | 0.1 |
| Cholesterol (mg) | 20 | 20 | 20 | 20 |
| Castor oil (g) | 0.4 | 0.4 | 0.4 | 0.4 |
| Cottonseed oil (g) | 0.6 | 0.6 | 0.6 | 0.6 |
| Ethanol (EtOH) | 0 | 0.05 | 0.1 | 0.15 |

**Test Example 10. Evaluation of viscosity and ability of sustained-release lipid pre-concentrate including goserelin as drug to form liquid crystals in aqueous phase**

The sustained-release lipid pre-concentrate prepared in Preparation Example 7 was filled into a 1-ml syringe and injected into 5 ml of a phosphate buffer (PBS) with pH 7.4 through a 23 gauge injection needle.

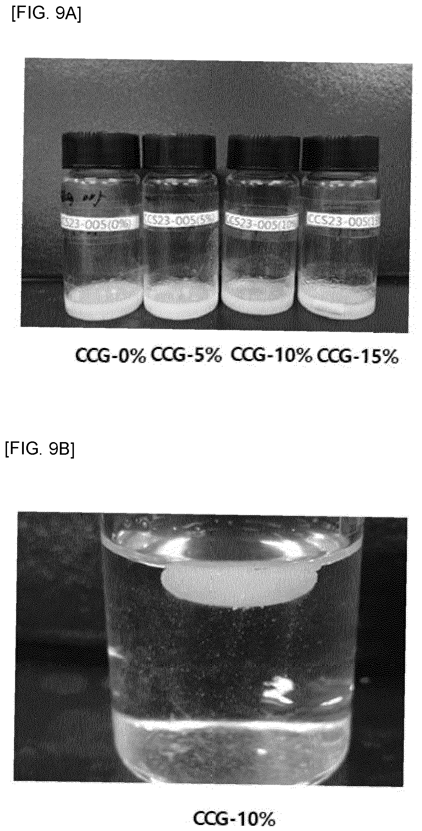

FIG 9 shows a set of photographs of sustained-release lipid pre-concentrates prepared in Preparation Example 7 being injected into PBS at pH 7.4.

As shown in FIG 9, it can be confirmed that liquid crystals are formed even when goserelin is used as a drug.

That is, it can be confirmed that the sustained-release lipid pre-concentrate according to the present invention forms liquid crystals regardless of the type of drug.

**Preparation Example 8. Preparation of sustained-release lipid pre-concentrate including semaglutide as drug**

Sustained-release lipid pre-concentrates including semaglutide as a drug were prepared according to the components and contents in the following Table 10.

The sustained-release lipid pre-concentrate was prepared as in the preparation method of Preparation Example 1.

[Table 10]

|  | Preparation Example 8-1 | Preparation Example 8-2 |
| --- | --- | --- |
| Semaglutide (g) | 0.1 | 0.1 |
| Cholesterol (mg) | 100 | 100 |
| Castor oil (g) | 1 | 1 |
| Cottonseed oil (g) | 1 | 1 |
| Ascorbic palmitate (ascorbic palmitate) (mg) | 100 | 100 |
| N-methylpyrrolidone (g) | 1 | |
| Glacial acetic acid (Acetic acid) (g) | | 0.1 |

**Test Example 11. Evaluation of long-term elution of sustained-release lipid pre-concentrate including semaglutide as drug**

900 ml of a phosphate buffer with pH 7.4 was put into an elutor, sampling was performed for 28 days by rotating the elutor at 50 rpm at 37.5°C by the USP I basket method, and then the samples were analyzed by HPLC under the following conditions.

<HPLC conditions>

Column: CAPCELL PAK $C_8$ (4.6 x 250 mm, 5.0 $\mu$m)
Mobile phase: MP A - 0.1 % TFA:Acetonitrile = 90:10 (v/v, %)
MP B - 0.1 % TFA:Acetonitrile = 10:90 (v/v, %)
Isocratic MP A (%):MP B (%) = 55:45
Flow rate: 0.8 ml/min
Temperature: 30°C
Injection amount: 6 $\mu$l
Wavelength: 200 nm (UV absorbance spectrophotometer)

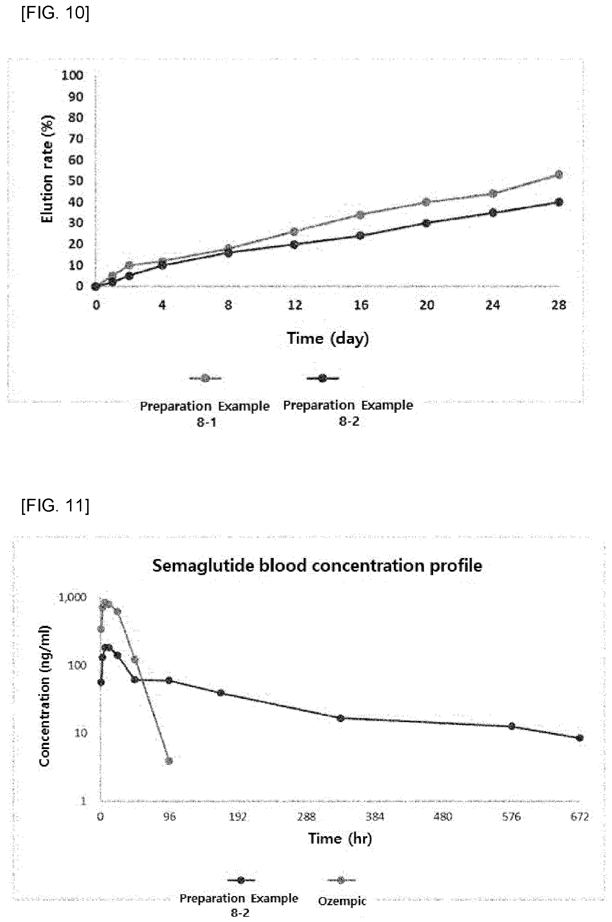

FIG 10 shows the results of evaluating the elution of sustained-release lipid pre-concentrates prepared in Preparation Example 8.
As described above, the elution evaluation results were obtained by performing *in vitro* release tests, and it can be confirmed that the sustained-release lipid pre-concentrate prepared according to the present invention showed a long-term effective sustained-release pattern.

**Test Example 12. Confirmation of initial release control and sustained-release effect *in vivo***

Experiments were performed to confirm the drug release behavior of sustained-release lipid pre-concentrates *in vivo.*
The sustained-release lipid pre-concentrate prepared according to Preparation Example 8-2 and an Ozempic strain (0.4 mg/kg as semaglutide) manufactured by Novo Nordisk as a control drug were subcutaneously administered to the backs of five 8-week-old SD rats (male) weighing an average of 300 g, and then blood was collected at 0, 1, 3, 6, 12, 24, 4896, 168, 336, 576, and 672 hours.
Thereafter, the concentration of semaglutide in the blood at each time point was measured using enzyme-linked immunosorbent assay (ELISA) in plasma samples from SD rats. In this case, GLP-1 (active) ELISA (IBL, Germany) was used as a kit.
FIG 11 shows changes in concentration of semaglutide over time. FIG 11 describes the average values for the five mice used in the experiment.
As shown in FIG 11, it can be confirmed that the maximum blood concentration (Cmax) of the physiologically active material in the sustained-release lipid pre-concentrate containing semaglutide (Preparation Example 8-2) was reduced 4.6-fold compared to an Ozempic strain, which is a control drug, and the blood concentration of the drug was maintained for 28 days.

That is, it can be confirmed that the sustained-release lipid pre-concentrate according to the present invention exhibited an initial release control effect and exhibited an excellent sustained-release effect for 28 days.

[Industrial Applicability]

The sustained-release lipid pre-concentrate according to the present invention forms liquid crystals when exposed to an aqueous medium, thus enabling the sustained-release of a drug, and can sustain drug release for two weeks to one month. In addition, the formed liquid crystals are sustainably released even at low viscosity, and have excellent ease of use due to low extrusion force when injected.

**Claims**

1. A sustained-release lipid pre-concentrate, which forms liquid crystals in an aqueous medium, comprising: a) a vegetable oil comprising an unsaturated fatty acid having 14 to 20 carbon atoms; and
b) one or more compounds selected from the group consisting of cholesterol and a sugar alcohol.

2. The sustained-release lipid pre-concentrate of claim 1, wherein component a) is one or more selected from the group consisting of castor oil, safflower oil, cottonseed oil, almond oil, avocado oil, canola oil, coconut oil, corn oil, flaxseed oil, linseed oil, macadamia oil, mustard oil, olive oil, palm oil, peanut oil, pumpkin seed oil, rice bran oil, sesame oil, soybean oil, sunflower oil, tea seed oil and walnut oil.

3. The sustained-release lipid pre-concentrate of claim 1, wherein the content of component a) is 50 to 98 parts by weight based on the total weight of the sustained-release lipid pre-concentrate.

4. The sustained-release lipid pre-concentrate of claim 1, wherein the sugar alcohol is one or more selected from the group consisting of mannitol, sorbitol, xylitol, erythritol, maltitol and lactitol.

5. The sustained-release lipid pre-concentrate of claim 1, wherein the content of component b) is 2 to 50 parts by weight based on the total weight of the sustained-release lipid pre-concentrate.

6. The sustained-release lipid pre-concentrate of claim 1, further comprising a drug, wherein the drug is one or more selected from the group consisting of nafamostat, doxycycline, liraglutide, semaglutide, lanreotide and goserelin.

7. The sustained-release lipid pre-concentrate of claim 6, wherein the content of the drug is 1 to 80 parts by weight based on 100 parts by weight of component a).

8. The sustained-release lipid pre-concentrate of claim 1, further comprising a stabilizer, wherein the stabilizer is one or more selected from the group consisting of sodium ascorbate (ViC_Na), ascorbic palmitate (ViC_palmitate), butylated hydroxyanisole (BHA), butylated hydroxytoluene (BHT), methionine, monothioglycerol, sodium thiosulfate and sodium metabisulfite.

9. The sustained-release lipid pre-concentrate of claim 8, wherein the content of the stabilizer is 0.1 to 10 parts by weight based on 100 parts by weight of component a).

10. The sustained-release lipid pre-concentrate of claim 1, wherein viscosity is 30 to 650 cP, and injection force is 1 to 50 N.

[FIG. 1]

**Preparation Example 1-1**

**Preparation Example 1-2**

**Preparation Example 1-3**

**Preparation Example 1-4**

[FIG. 2A]

[FIG. 2B]

[FIG. 3A]

Preparation Example 3-1    Preparation Example 3-2    Preparation Example 3-2

Preparation Example 3-4    Preparation Example 3-5    Preparation Example 3-6

[FIG. 3B]

[FIG. 4]

[FIG. 5]

Peroxide value of safflower oil according to antioxidant for harsh 2 weeks at 60°C

Peroxide value of castor oil according to antioxidant for harsh 2 weeks at 60°C

Peroxide value of cottonseed oil according to antioxidant for harsh 2 weeks at 60°C

Peroxide value of oleic acid according to antioxidant for harsh 2 weeks at 60°C

EP 4 321 152 A1

[FIG. 6]

[FIG. 7]

20

[FIG. 8A]

CCS-0%   CCS-5%   CCS-10%   CCS-15%

[FIG. 8B]

CCS-0%   CCS-5%  CCS-10% CCS-15%

[FIG. 9A]

CCG-0% CCG-5% CCG-10% CCG-15%

[FIG. 9B]

CCG-10%

[FIG. 10]

[FIG. 11]

## INTERNATIONAL SEARCH REPORT

| International application No. |
| --- |
| **PCT/KR2022/003912** |

### A. CLASSIFICATION OF SUBJECT MATTER

**A61K 9/08**(2006.01)i; **A61K 9/06**(2006.01)i; **A61K 9/00**(2006.01)i; **A61K 47/12**(2006.01)i; **A61K 47/28**(2006.01)i; **A61K 47/26**(2006.01)i; **A61K 47/44**(2006.01)i; **A61K 31/24**(2006.01)i; **A61K 38/26**(2006.01)i

According to International Patent Classification (IPC) or to both national classification and IPC

### B. FIELDS SEARCHED

Minimum documentation searched (classification system followed by classification symbols)

A61K 9/08(2006.01); A61K 47/14(2006.01); A61K 47/30(2006.01); A61K 9/107(2006.01); A61K 9/22(2006.01); A61K 9/52(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 서방성 제제(sustained-release concentrate), 전구 제제(pre concentrate), 지질(lipid), 주입력(injectability), 점도(viscosity)

### C. DOCUMENTS CONSIDERED TO BE RELEVANT

| Category* | Citation of document, with indication, where appropriate, of the relevant passages | Relevant to claim No. |
| --- | --- | --- |
| X | KR 10-2013-0101051 A (NOVAGALI PHARMA SA) 12 September 2013 (2013-09-12) See abstract; claims 1-2, 5 and 7; paragraphs [0033], [0061] and [0126]-[0127]; and figure 1. | 1-7,10 |
| Y | | 8-9 |
| Y | KR 10-2004-0066191 A (TAKEDA PHARMACEUTICAL COMPANY LIMITED) 23 July 2004 (2004-07-23) See page 14, seventh paragraph. | 8-9 |
| A | KR 10-2003-0011856 A (ITALFARMACO S.P.A.) 11 February 2003 (2003-02-11) See entire document. | 1-10 |
| A | KR 10-2011-0050540 A (AMYLIN PHARMACEUTICALS, INC.) 13 May 2011 (2011-05-13) See entire document. | 1-10 |

☑ Further documents are listed in the continuation of Box C.  ☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **22 June 2022** | **22 June 2022** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

| INTERNATIONAL SEARCH REPORT | International application No. |
|---|---|
| | **PCT/KR2022/003912** |

**C.    DOCUMENTS CONSIDERED TO BE RELEVANT**

| Category* | Citation of document, with indication, where appropriate, of the relevant passages | Relevant to claim No. |
|---|---|---|
| A | KR 10-2004-0018434 A (ASTELLAS PHARMA INC.) 03 March 2004 (2004-03-03)<br>See entire document. | 1-10 |

**INTERNATIONAL SEARCH REPORT**
Information on patent family members

International application No.

**PCT/KR2022/003912**

| Patent document cited in search report | | | Publication date (day/month/year) | Patent family member(s) | | | Publication date (day/month/year) |
|---|---|---|---|---|---|---|---|
| KR | 10-2013-0101051 | A | 12 September 2013 | BR | 112013004570 | A2 | 06 September 2016 |
| | | | | CA | 2809460 | A1 | 08 March 2012 |
| | | | | CA | 2809460 | C | 11 December 2018 |
| | | | | CN | 103140216 | A | 05 June 2013 |
| | | | | CN | 103140216 | B | 15 July 2015 |
| | | | | DK | 2425814 | T3 | 08 September 2013 |
| | | | | EA | 028375 | B1 | 30 November 2017 |
| | | | | EA | 201300314 | A1 | 29 November 2013 |
| | | | | EA | 201300314 | A8 | 31 August 2017 |
| | | | | EP | 2425814 | A1 | 07 March 2012 |
| | | | | EP | 2425814 | B1 | 19 June 2013 |
| | | | | EP | 2611414 | A1 | 10 July 2013 |
| | | | | EP | 2611414 | B1 | 12 November 2014 |
| | | | | JP | 2013-536829 | A | 26 September 2013 |
| | | | | JP | 5829685 | B2 | 09 December 2015 |
| | | | | MX | 2013002115 | A | 28 June 2013 |
| | | | | MY | 157513 | A | 15 June 2016 |
| | | | | NZ | 607103 | A | 25 September 2015 |
| | | | | US | 2012-0058187 | A1 | 08 March 2012 |
| | | | | US | 2013-0164285 | A1 | 27 June 2013 |
| | | | | US | 2016-0331683 | A1 | 17 November 2016 |
| | | | | US | 9107822 | B2 | 18 August 2015 |
| | | | | WO | 2012-028733 | A1 | 08 March 2012 |
| KR | 10-2004-0066191 | A | 23 July 2004 | CA | 2471521 | A1 | 10 July 2003 |
| | | | | CA | 2471521 | C | 02 November 2010 |
| | | | | CN | 101444476 | A | 03 June 2009 |
| | | | | CN | 101444476 | B | 30 November 2011 |
| | | | | CN | 1620285 | A | 25 May 2005 |
| | | | | CN | 1620285 | C | 25 May 2005 |
| | | | | EP | 1466596 | A1 | 13 October 2004 |
| | | | | EP | 1466596 | B1 | 31 August 2016 |
| | | | | EP | 1466596 | B8 | 11 January 2017 |
| | | | | JP | 2003-252751 | A | 10 September 2003 |
| | | | | US | 2005-0064039 | A1 | 24 March 2005 |
| | | | | WO | 03-055470 | A1 | 10 July 2003 |
| KR | 10-2003-0011856 | A | 11 February 2003 | CA | 2409854 | A1 | 29 November 2001 |
| | | | | CA | 2409854 | C | 08 February 2011 |
| | | | | CN | 100479856 | C | 22 April 2009 |
| | | | | CN | 1430502 | A | 16 July 2003 |
| | | | | CZ | 20023772 | A3 | 12 February 2003 |
| | | | | EP | 1283700 | A2 | 19 February 2003 |
| | | | | EP | 1283700 | B1 | 29 March 2006 |
| | | | | HR | 20020929 | A2 | 29 February 2004 |
| | | | | HR | P20020929 | A2 | 29 February 2004 |
| | | | | JP | 2003-534265 | A | 18 November 2003 |
| | | | | JP | 4954423 | B2 | 13 June 2012 |
| | | | | US | 2003-0171299 | A1 | 11 September 2003 |
| | | | | US | 7157099 | B2 | 02 January 2007 |
| | | | | WO | 01-89479 | A2 | 29 November 2001 |
| | | | | WO | 01-89479 | A3 | 28 March 2002 |

Form PCT/ISA/210 (patent family annex) (July 2019)

**INTERNATIONAL SEARCH REPORT**
Information on patent family members

International application No.

**PCT/KR2022/003912**

| Patent document cited in search report | | | Publication date (day/month/year) | Patent family member(s) | | | Publication date (day/month/year) |
|---|---|---|---|---|---|---|---|
| KR | 10-2011-0050540 | A | 13 May 2011 | BR | PI0918904 | A2 | 01 December 2015 |
| | | | | BR | PI0918904 | B1 | 13 October 2020 |
| | | | | BR | PI0918904 | B8 | 25 May 2021 |
| | | | | CA | 2734525 | A1 | 11 March 2010 |
| | | | | CA | 2734525 | C | 26 July 2016 |
| | | | | CN | 102164597 | A | 24 August 2011 |
| | | | | CN | 102164597 | B | 03 September 2014 |
| | | | | CN | 104248623 | A | 31 December 2014 |
| | | | | CN | 104248623 | B | 03 April 2020 |
| | | | | EP | 2341905 | A1 | 13 July 2011 |
| | | | | EP | 2341905 | B1 | 29 April 2020 |
| | | | | EP | 3685837 | A1 | 29 July 2020 |
| | | | | JP | 2012-502056 | A | 26 January 2012 |
| | | | | JP | 2015-110637 | A | 18 June 2015 |
| | | | | JP | 5744735 | B2 | 08 July 2015 |
| | | | | JP | 6051243 | B2 | 27 December 2016 |
| | | | | US | 2011-0212138 | A1 | 01 September 2011 |
| | | | | US | 2015-0056285 | A1 | 26 February 2015 |
| | | | | US | 2016-0346357 | A1 | 01 December 2016 |
| | | | | US | 2018-0271946 | A1 | 27 September 2018 |
| | | | | US | 2020-0405815 | A1 | 31 December 2020 |
| | | | | US | 8895033 | B2 | 25 November 2014 |
| | | | | WO | 2010-028257 | A1 | 11 March 2010 |
| KR | 10-2004-0018434 | A | 03 March 2004 | CA | 2448814 | A1 | 23 January 2003 |
| | | | | CN | 1523998 | A | 25 August 2004 |
| | | | | EP | 1413310 | A1 | 28 April 2004 |
| | | | | US | 2004-0142043 | A1 | 22 July 2004 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5480656 A
- WO 2005117830 A

- KR 101494594